# EUROPÄISCHE PATENTSCHRIFT

(11) **EP 0 265 694 B1**
(45) Veröffentlichungstag und Bekanntmachung des Hinweises auf die Patenterteilung: **22.12.1993**
(21) Anmeldenummer: 87114259.2
(22) Anmeldetag: 30.09.1987
(51) Int. Cl.: A61B 5/04

(54) **Herzdiagnosegerät**
Cardiac diagnostic device
Dispositif de diagnostic cardiaque

(30) Priorität: 17.10.1986 DE 3635346
(43) Veröffentlichungstag der Anmeldung: 04.05.1988
(73) Patentinhaber: Uhlemann, Hans-Joachim, D-45701 Herten (DE)
(72) Erfinder: Uhlemann, Hans-Joachim, D-45701 Herten (DE)
(74) Vertreter: von Rohr, Hans Wilhelm, Dipl.-Phys.

(56) Entgegenhaltungen:
- EP-A- 0 027 974
- EP-A- 0 170 448
- EP-A- 0 178 990
- EP-A- 0 207 678
- DE-A- 3 409 094
- FR-A- 1 571 024
- FR-A- 1 571 024
- GB-A- 2 142 727
- US-A- 4 350 164
- PATENT ABSTRACTS OF JAPAN, Band 7, Nr. 30 (P-173)[1175], 5. Februar 1983; & JP-A-57 182 184 (KUNIO TAMURA) 09-11-1982

## Beschreibung

Die Erfindung betrifft ein Herzdiagnosegerät nach dem Oberbegriff von Anspruch 1.

Das bekannte Herzdiagnosegerät, von dem die Erfindung ausgeht (EP-A1 0 178 990) ist für den Notfall besonders geeignet, nämlich ortsunabhängig. Mit seinen Aufnahmeelektroden ist das Herzdiagnosegerät im Notfall unmittelbar auf einen Körperbereich eines Patienten kontaktierend aufsetzbar. Das Gehäuse dieses Herzdiagnosegeräts hat eine rechteckige, plattenförmige Gestalt, wobei auf der Unterseite des Gehäuses die Aufnahmeelektroden angebracht sind und auf der gegenüberliegenden Oberseite des Gehäuses die Graphikanzeigeeinheit bzw. ein entsprechender Bildschirm sowie verschiedene Einstellelemente und optische und akustische Anzeigeelemente angeordnet sind. Bei diesem Herzdiagnosegerät ist die Auflösung der Graphikanzeigeeinheit einstellbar. Dabei ist notwendigerweise auch eine Referenzanzeige vorzusehen, die man entweder auf die Nullinie des EKG justieren oder in einem Freibereich der Graphikanzeigeeinheit anordnen kann. Besonders zweckmäßig ist es, daß bei diesem Herzdiagnosegerät ein auf der Graphikanzeigeeinheit angezeigtes EKG wahlweise festhaltbar ist, daß also die Anzeige eingefroren werden kann. Das erfolgt durch Betätigung eines der Einstellelemente auf der Oberseite des Gehäuses bei noch am Körper des Patienten befindlichem Herzdiagnosegerät. Mit einem weiteren Einstellelement läßt sich eine eingefrorene Anzeige wieder löschen. Man kann also das Herzdiagnosegerät vom Körper des Patienten nach Einfrieren der Anzeige abnehmen und unter besserer Beleuchtung oder besserer Zugänglichkeit dann die Anzeige des EKG auf der Graphikanzeigeeinheit betrachten. Im übrigen ist am Gehäuse ein elektrischer Anschluß für ein Übertragungskabel vorgesehen.

Das bekannte, zuvor erläuterte Herzdiagnosegerät ist für eine weite Verbreitung bei allen niedergelassenen Ärzten noch nicht optimal geeignet, da das relativ großflächige, plattenartige Gehäuse zu voluminös ist und dementsprechend viel Platz in einer Arzttasche od. dgl. einnimmt. Die Folge ist, daß dieses Herzdiagnosegerät von einem normalen praktischen Arzt, der Hausbesuche macht, normalerweise nicht mitgenommen werden wird.

Im übrigen ist ein Notfalldiagnosegerät bekannt (US-PS 4,350,164) das von jedem Arzt in einer Arzttasche oder Jackentasche ohne weiteres mitgeführt werden kann, da sein Gehäuse röhrenförmig ausgeführt ist. Dieses Diagnosegerät ergibt aber nur eine minimale diagnostische Aussage, nämlich nur eine Aussage Ober die Herzfrequenz mittels einer blinkenden Indikatorleuchte. Nur mit zusätzlichem konstruktiven Aufwand und unter Verzicht auf eine einfache und robuste Konstruktion kann im übrigen erreicht werden, daß der Abstand der hier an der Stirnseite des Gehäuses konzentrierten Aufnahmeelektroden ausreichend groß ist. Gerade dieser Abstand aber ist eine wesentliche Voraussetzung für eine umfassende und zuverlässige diagnostische Aussage. Dieses Diagnosegerät ist also in der diagnostischen Leistungsfähigkeit mit dem eingangs erläuterten Herzdiagnosegerät, von dem die Erfindung ausgeht, nicht vergleichbar.

Der Erfindung liegt nun die Aufgabe zugrunde, daß bekannte Herzdiagnosegerät unter Beibehaltung seiner diagnostischen Leistungsfähigkeit so zu gestalten, daß es von jedem Arzt in einer Arzttasche oder Jackentasche ohne weiteres mitgeführt werden kann.

Die zuvor aufgezeigte Aufgabe ist bei einem Herzdiagnosegerät mit den Merkmalen des kennzeichnenden Teils des Anspruchs 1 gelöst. Erfindungsgemäß ist erkannt worden, daß eine langgestreckte Graphikanzeigeeinheit ohne weiteres auch an einem röhrenförmigen Gehäuse anbringbar ist. Weiter ist erkannt worden, daß die Anordnung der Aufnahmeelektroden an einer Längsseite des Gehäuses nahe der vorderen und der hinteren Stirnseite auch bei einem röhrenförmigen Gehäuse beibehalten werden kann und daß dadurch von selbst der gewünschte große Abstand der Aufnahmeelektroden voneinander gewährleistet ist. Schließlich ist erkannt worden, daß ein bei einem röhrenförmigen, etwa füllhalterartig geformten Gehäuse zur dauernden Mitführung besonders zweckmäßiger Halteclip, der wie üblich von einem Ende des Gehäuses ausgeht, gleichzeitig eine der Aufnahmeelektroden sein kann. Durch diese geschickte Gestaltung des erfindungsgemäßen Herzdiagnosegeräts kann dieses nun praktisch von jedem Arzt ohne weiteres jederzeit mitgeführt werden. Das Herzdiagnosegerät läßt sich im Arztkoffer oder sogar in der Jackentasche eines Arztes nicht wesentlich anders unterbringen als ein normaler Füllhalter od. dgl..

Es gibt eine Vielzahl von Möglichkeiten, das erfindungsgemäße Herzdiagnosegerät auszugestalten und weiterzubilden, wozu einerseits auf die dem Anspruch 1 nachgeordneten Ansprüche, andererseits auf die nachfolgende Erläuterung dieser Ansprüche sowie Erläuterung bevorzugter Ausführungsbeispiele der Erfindung anhand der Zeichnung verwiesen werden darf.

In der Zeichnung zeigt
- Fig. 1: in perspektivischer Ansicht ein erstes Ausführungsbeispiel eines Herzdiagnosegeräts,
- Fig. 2: eine Ansicht des Herzdiagnosegeräts aus Fig. 1 von unten in Fig. 1 gesehen,
- Fig. 3: in Fig. 1 entsprechender Darstellung ein zweites Ausführungsbeispiel eines Herzdiagnosegeräts,
- Fig. 4: in Fig. 2 entsprechender Darstellung eine Ansicht des Herzdiagnosegeräts aus Fig. 3 und
- Fig. 5: in Stirnansicht, in Fig. 3 von rechts gesehen, das Herzdiagnosegerät aus Fig. 3.

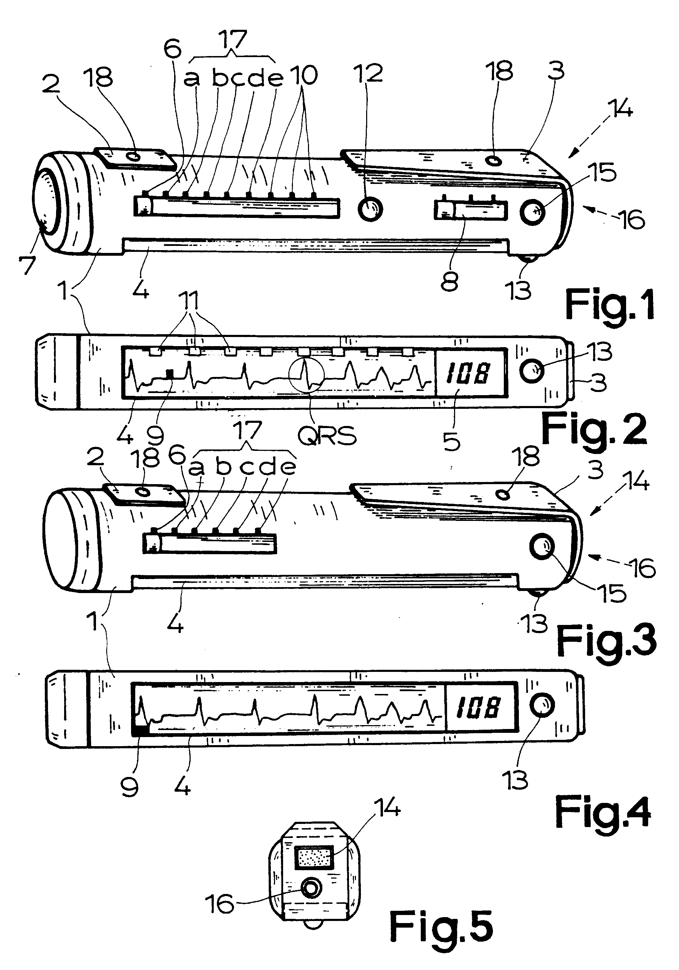

Das in Fig. 1 in einem ersten Ausführungsbeispiel dargestellte, vollintegrierte Herzdiagnosegerät weist ein Gehäuse 1 und eine in dem Gehäuse 1 angeordnete, in der Zeichnung nicht erkennbare Diagnoseelektronik auf. An dem Gehäuse 1 angeordnet sind zwei Aufnahmeelektroden 2, 3, die elektrisch mit der Diagnoseelektronik im Gehäuse verbunden sind. Durch Aufsetzen des Gehäuses auf einen Körperbereich eines Patienten sind die Aufnahmeelektroden 2, 3 an die Haut des Patienten kontaktierend andrückbar und dann sind über die Aufnahmeelektroden 2, 3 und die Diagnoseelektronik die Herzfrequenz, ein Elektrokardiogramm (EKG), die QRS-Durchgänge eines EKG, mit einer entsprechenden Vorverstärkung aber auch ein EEG od. dgl. aufnehmbar.

In dem Gehäuse 1 sind weiter eine hier nicht dargestellte Spannungsversorgung, insbesondere eine Trockenbatterie oder ein aufladbarer Akkumulator, eine flächige, dünne Graphikanzeigeeinheit 4, vorzugsweise und hier verwirklicht eine optische Herzfrequenzanzeigeeinheit 5 und ein Ein/Aus-Schalter 6 angeordnet und elektrisch mit der Diagnoseelektronik verbunden. Die Diagnoseelektronik umfaßt dementsprechend ferner einerseits eine Auswerteelektronik, andererseits eine Ansteuerelektronik für die Graphikanzeigeeinheit 4 und die hier ebenfalls vorgesehene Herzfrequenzanzeigeeinheit 5. Wie Fig. 1 dabei zeigt, ist das Gehäuse 1 langgestreckt, röhrenförmig ausgeführt. In Anpassung an diese Form des Gehäuses 1 sind zwei Aufnahmeelektroden 2, 3 an einer Längsseite des Gehäuses 1 nahe der vorderen bzw. hinteren Stirnseite angeordnet und ist die Graphikanzeigeeinheit 4 an einer weiteren Längsseite des Gehäuses 1 gegenüber den Aufnahmeelektroden 2, 3 angeordnet. Dabei erstreckt sich die Graphikanzeigeeinheit 4 auf der entsprechenden Längsseite des Gehäuses 1 über einen wesentlichen Teil der Länge des Gehäuses 1. Damit ist gewährleistet, daß für die Graphikanzeigeeinheit 4 ein optimaler Platz gegeben ist, ferner ist gewährleistet, daß die Aufnahmeelektroden 2, 3 einen ausreichend großen Abstand voneinander haben, um meßtechnisch zuverlässige Ergebnisse zu liefern. Schließlich ist diese Ausgestaltung des Gehäuses 1 und Anordnung der Aufnahmeelektroden 2, 3 und der Graphikanzeigeeinheit 4 auch handhabungstechnisch zweckmäßig, da die Graphikanzeigeeinheit 4 so besonders leicht abgelesen werden kann. Die Fig. 1 macht deutlich, daß das Gehäuse 1 etwa füllhalterartig geformt ist und eine Aufnahmeelektrode 3 die Form eines üblichen, von einem Ende des Gehäuses 1 ausgehenden Halteclips hat.

Das in Fig. 1 dargestellte Herzdiagnosegerät läßt sich praktisch wie eine an sich bekannte, von Ärzten schon heute stets mitgeführte Diagnoseleuchte handhaben. Es hat sich dabei gezeigt, daß es ausreicht, wenn das Gehäuse 1 eine Länge von 100 mm bis 160 mm, insbesondere von ca. 130 mm, und einen Durchmesser bzw. einen maximalen Durchmesser von 10 mm bis 40 mm, insbesondere von ca. 16 mm, aufweist.

Bevorzugt ist eine Ausgestaltung der Graphikanzeigeeinheit 4 als LCD-Graphikanzeige, beispielsweise bekannt als LCD-Graphic-Display der Firma Hitachi Ltd.. Die Graphikanzeigeeinheit 4 könnte aber auch als heutzutage schon am Markt erhältlicher Flachbildschirm ausgeführt sein bzw. aus einer Reihe von nebeneinander angeordneten Flachbildschirmen zusammengesetzt sein, um eine erforderliche Mindestlänge zu haben. Hinsichtlich der Herzfrequenzanzeigeeinheit 5 zeigt Fig. 2, daß diese eine LCD-Ziffernanzeige sein kann. Auch eine LED-Ziffernanzeige kommt in Frage, hat aber einen höheren Stromverbrauch. Positiv ist dabei, daß eine LED-Ziffernanzeige auch bei Dunkelheit erkennbar ist. Jedenfalls empfiehlt es sich, wie in Fig. 2 und Fig. 4 dargestellt, die Herzfrequenzanzeigeeinheit 5 in die Graphikanzeigeeinheit 4 zu integrieren.

Bei der begrenzten Größe der Graphikanzeigeeinheit 4 des Herzdiagnosegeräts ist es zweckmäßig, die Auflösung der Graphikanzeigeeinheit 4 einstellen zu können. Zunächst ist es dabei möglich, daß die Auflösung der Graphikanzeigeeinheit 4 mittels eines Auflösungswahlschalters 8 einstellbar ist, vorzugsweise in den Stufen 0,5 mV/cm, 1 mV/cm, 3 mV/cm. In Fig. 1 ist dieser Auflösungswahlschalter 8 mit seinen drei Stellungen, dort eingestellt auf die Stufe 0,5 mV/cm, gezeigt. Für eine EEG-Aufnahme gilt, daß hier mit dem Herzdiagnosegerät lediglich eine Existenz von Gehirnströmen nachgewiesen werden kann, und zwar bei entsprechender Vorverstärkung des Eingangssignals (Vorverstärkung etwa 1.000-fach). Eine qualitative Gehirnstrommessung kann wertvolle zusätzliche Aufschlüsse hinsichtlich des Zustands eines Patienten geben.

Alternativ zu der manuellen Einstellbarkeit der Auflösung der Graphikanzeigeeinheit 4, aber auch zusätzlich dazu kann die Auflösung der Graphikanzeigeeinheit 4 auch automatisch und vorzugsweise im wesentlichen stufenlos nach Maßgabe der maximalen Amplitude der Herzaktion einstellbar sein. Eine derartige automatische Auflösungsnachführung ist mit modernen regelungstechnischen Verfahren ohne weiteres möglich (Lock-In-Verfahren). Diese Alternative ist in Fig. 3 der Zeichnung verwirklicht.

Ist die Auflösung der Graphikanzeigeeinheit 4 einstellbar, so ist es zweckmäßig, in der Graphikanzeigeeinheit 4 selbst eine der gewählten bzw. gegebenen Auflösung entsprechende Referenzanzeige 9 vorzusehen. Das muß nicht heißen, daß diese Referenzanzeige 9 separat vorhanden ist, sondern es ist beispielsweise bei einer LCD-Graphikanzeige so, daß durch eine bestimmte Ansteuerung der Graphikanzeige auf der Graphikanzeigeeinheit 4 eine Referenzanzeige 9 erscheint. Diese Referenzanzeige 9 ist hier ein 1 mV-Block in gut erkennbarer Darstellung.

Fig. 2 macht deutlich, daß die Referenzanzeige 9 auf die Nullinie des EKG bzw. EEG justiert ist. Fig. 4 macht im Gegensatz dazu deutlich, daß dort die Referenzanzeige 9 in einem Freibereich der Graphikanzeigeeinheit 4, nämlich in einem Winkel der Graphikanzeigeeinheit 4 angeordnet ist. Jedenfalls erlaubt es die Referenzanzeige 9, die absolute Größe der Spitzen im EKG unabhängig von der manuell oder automatisch gewählten Auflösung der Graphikanzeigeeinheit 4 zu bestimmen. Das gibt wertvolle Aufschlüsse für den behandelnden Arzt.

Ein wesentlicher Aspekt in der Ausgestaltung des Herzdiagnosegeräts besteht darin, daß man die Anzeige der Graphikanzeigeeinheit 4 bzw. der Herzfrequenzanzeigeeinheit 5 einfrieren können sollte. Zum Festhalten eines auf der Graphikanzeigeeinheit 4 angezeigten EKG kann ein manuell betätigbarer Halteschalter 10 vorgesehen sein. Wird der Halteschalter 10 betätigt, so wird der Spannungszustand an der Graphikanzeigeeinheit 4 unverändert beibehalten. Nimmt nun der behandelnde Arzt das Herzdiagnosegerät vom Patienten ab, so kann er das größenmäßig begrenzte EKG auf der Graphikanzeigeeinheit 4 eindeutiger und eingehender analysieren. Der Halteschalter 10 sollte zweckmäßigerweise eine Nullstellung, eine Haltestellung und eine Löschstellung aufweisen, wie in Fig. 1 im dort gezeigten Ausführungsbeispiel angedeutet.

Fig. 3 zeigt ein Ausführungsbeispiel, bei dem das Festhalten eines auf der Graphikanzeigeeinheit 4 angezeigten EKG automatisch erfolgt, und zwar bei Abnahme der Aufnahmeelektroden 2, 3 von der Haut eines Patienten nach einer durchgeführten Aufnahme. Hier kann entsprechend dann das Wiederaufsetzen der Aufnahmeelektroden 2, 3 auf die Haut eine Löschung des festgehaltenen EKG und eine Fortführung der Aufnahme auslösen. Besonders zweckmäßig ist es, wenn als Auslösekriterium der Bahnwiderstand zwischen den Aufnahmeelektroden 2, 3 dient. Versuche haben gezeigt, daß es zweckmäßig ist, das Auslösekriterium Bahnwiderstand so zu wählen, daß bei einem Bahnwiderstand unter 3 kΩ eine Aufnahme des EKG und über 3 kΩ ein Festhalten des EKG erfolgt.

Zweckmäßig ist es, wenn die Diagnoseelektronik einen Datenspeicher aufweist und ein auf der Graphikanzeigeeinheit 4 angezeigtes EKG darin abspeicherbar ist. Besonders empfiehlt es sich, daß der Datenspeicher platzmäßig auf die Speicherung mehrerer EKG ausgelegt ist. Im Gegensatz zum "Einfrieren" der Anzeige, das zuvor erläutert worden ist, jedoch nur eine einzige Momentaufnahme eines EKG darstellt, erlaubt es die Speicherung auf elektronischem Wege, mehrere Momentaufnahmen festzuhalten, beispielsweise jeweils in Momenten charakteristischer Veränderungen des EKG. Um feststellbar zu machen, welcher Speicher belegt ist und welcher Speicher noch frei ist, läßt sich mit Speicheranzeigen arbeiten. Insoweit zeigt Fig. 2, daß in der Graphikanzeigeeinheit 4 eine Speicheranzeige 11 mit mehreren Anzeigestufen vorgesehen bzw. ansteuerbar ist und daß mit Hilfe der Speicheranzeige 11 feststellbar ist, ob und bejahendenfalls welcher Speicher bereits belegt ist.

Für die Steuerung der Speicherung eines auf der Graphikanzeigeeinheit 4 angezeigten EKG reicht ein Speicherauslöseschalter 12 aus, der in Fig. 1 der Zeichnung dargestellt ist und dort die Form eines Druckschalters hat. In ähnlicher Weise wie beim Einfrieren der Anzeige der Graphikanzeigeeinheit 4 läßt sich auch die elektronische Speicherung automatisch dadurch auslösen, daß das Gehäuse 1 mit den Aufnahmeelektroden 2, 3 vom Körper des Patienten abgenommen wird.

Schließlich empfiehlt es sich, daß der Inhalt des Speichers in der Diagnoseelektronik über einen angeschlossenen bzw. anschließbaren Streifenschreiber bzw. Streifendrucker ausdruckbar ist. Damit läßt sich gleichzeitig oder nachträglich eine vollständige Dokumentation eines Notfalls erstellen.

Wie Fig. 1 zeigt, ist es ohne weiteres möglich, in dem Gehäuse 1 des Herzdiagnosegeräts auch noch eine optische ORS-Anzeige 13, insbesondere in Form einer LED-Anzeige, vorzusehen und elektrisch mit der Diagnoseelektronik zu verbinden. Entsprechend gilt, daß es auch möglich ist, daß in dem Gehäuse 1 eine akustische QRS-Anzeige 14, insbesondere in Form eines Piezolautsprechers, vorgesehen und elektrisch mit der Diagnoseelektronik verbunden ist. Das ist in Fig. 5 zu erkennen, da die akustische QRS-Anzeige 14 an der der Diagnoseleuchte 7 fernen Stirnseite des Gehäuses 1 angeordnet ist. Optisch und/oder akustisch läßt sich so der Herzschlag eines Patienten deutlich wiedergeben. Weiter ist es zweckmäßig, daß in dem Gehäuse 1 eine optische und/oder akustische Alarmanzeige 15, insbesondere in Form einer LED-Anzeige bzw. eines Piezolautsprechers, vorgesehen und elektrisch mit der Diagnoseelektronik verbunden ist. In Fig. 1 und in Fig. 3 ist lediglich eine optische Alarmanzeige 15 in Form einer LED-Anzeige vorgesehen. Bei einer rein akustischen Alarmanzeige ist zweckmäßigerweise eine untere Grenze mit 50 Herzschlägen pro Minute und eine obere Grenze mit 150 Herzschlägen pro Minute fest eingestellt. Das ist für einen Notfall eine ausreichende Eingrenzung.

Zuvor ist ausgeführt worden, daß es zweckmäßig sein kann, das Herzdiagnosegerät extern an weitere Geräte, insbesondere an einen Streifenschreiber od. dgl. anzuschließen. Dazu und zur Aufladung eines aufladbaren Akkumulators empfiehlt es sich, in dem Gehäuse 1 einen elektrischen Anschluß 16 für ein Übertragungskabel od. dgl. vorzusehen. Dieser elektrische Anschluß 16 ist nach der Darstellung in Fig. 5 an einer Stirnseite des Gehäuses 1 anzuordnen, um die Längsseiten des Gehäuses 1, jedenfalls bei der langgestreckt-röhrenförmigen Gestaltung der Ausführungsbeispiele, nicht unnötig zu "besetzen". Im übrigen ist eine axiale Herausführung eines Übertragungskabels aus dem Anschluß 16 handhabungstechnisch zweckmäßiger als eine radiale.

Wie die Fig. 1 und 3 deutlich machen, ist im Gehäuse 1 neben dem Ein/Aus-Schalter 6 noch ein Funktionswahlschalter 17 zur Wahl der unterschiedlichen möglichen Funktionen des Herzdiagnosegeräts vorgesehen. In beiden dargestellten Ausführungsbeispielen ist eine Besonderheit dabei die, daß der Ein/Aus-Schalter 6 und der Funktionswahlschalter 17 miteinander als mehrstufiger Schalter integriert sind. Ein mehrstufiger Schalter könnte als Drehschalter ausgeführt sein, der bevorzugten, langgestreckten/röhrenförmigen Gestaltung des Gehäuses 1 entspricht es aber, daß erforderliche Schalter 6, 8, 10, 17 als Schiebeschalter ausgeführt und in Längsrichtung des Gehäuses 1 angeordnet sind.

Im in Fig. 1 dargestellten Ausführungsbeispiel eines komplett ausgestatteten Herzdiagnosegeräts hat der mit dem Ein/Aus-Schalter 6 integrierte Funktionswahlschalter 17 die Schaltstellungen Diagnoseleuchte,, EKG-optisch QRS, EKG-akustisch QRS, EKG-Graphikanzeige, EEG-Graphikanzeige (1.000-fach vorverstärkt). Im übrigen läßt Fig. 1 erkennen, daß im hier dargestellten und bevorzugten Ausführungsbeispiel auch der Halteschalter 10 noch mit dem Ein/Aus-Schalter 6 und dem Funktionswahlschalter 17 integriert ist.

Demgegenüber weist das Herzdiagnosegerät im Ausführungsbeispiel gemäß Fig. 3 lediglich einen Ein/Aus-Schalter 6 mit integriertem Funktionswahlschalter 17 auf. Da hier eine Speichermöglichkeit nicht vorgesehen ist, ist auch ein Speicherauslöseschalter nicht vorgesehen. Ein Auslösungswahlschalter fehlt, da lediglich eine automatische Auflösungsanpassung erfolgt. Schließlich fehlt hier eine Diagnoseleuchte.

Beiden Ausführungsbeispielen ist gemeinsam, daß die Aufnahmeelektroden 2, 3 mit Verbindungselementen zum Anschließen bzw. Anrasten von Dauerhaftelektroden, also Langzeitelektroden versehen sind. Diese Verbindungselemente sind in den dargestellten Ausführungsbeispielen als Rastöffnungen 18 ausgeführt, in die die kugelkopfähnlichen Rastelemente üblicher Dauerhaftelektroden, wie sie am Markt erhältlich sind, eingerastet werden können. Diese Maßnahme erlaubt es, das Herzdiagnosegerät nicht nur zur Notfalldiagnose kurzzeitig einzusetzen, sondern auch für eine längere Beobachtung eines Patienten zu verwenden. Insbesondere in Verbindung mit einem elektrischen Anschluß 16 für ein Übertragungskabel läßt sich das Notfall-Diagnosegerät über den Notfall hinaus auch im Krankenhaus an ein stationäres Gerät angeschlossen verwenden.

Zuvor ist gesagt worden, daß die zuvor erläuterte, bevorzugte Ausgestaltung des Herzdiagnosegerätes dazu führt, daß diese etwa wie eine Diagnoseleuchte eines Arztes gehandhabt werden kann. Wie Fig. 1 zeigt, ist es auch möglich, das Herzdiagnosegerät mit einer Diagnoseleuchte zu kombinieren, mit anderen Worten an einer Stirnseite des Gehäuses 1 eine Diagnoseleuchte 7 anzuordnen. Diese Kombination mit einer Diagnoseleuchte 7 hat einerseits den Vorteil, daß ein und dasselbe Gerät zwei ganz wesentliche Hilfsmittel eines Arztes im Notfalleinsatz miteinander vereint, daß andererseits die Leuchtkraft der Diagnoseleuchte 7 als Indiz dafür herangezogen werden kann, ob die Spannungsversorgung, also die Batterie oder der Akkumulator, noch eine ausreichende Spannung hat.

## Patentansprüche

1. Herzdiagnosegerät mit einem langgestreckten Gehäuse (1), einer in dem Gehäuse (1) angeordneten Diagnoseelektronik und zwei an einer Längsseite des Gehäuses (1) nahe der vorderen bzw. hinteren Stirnseite des Gehäuses (1) angeordneten, elektrisch mit der Diagnoseelektronik verbundenen Aufnahmeelektroden (2, 3), wobei in dem Gehäuse (1) weiter eine Spannungsversorgung, eine flächige, dünne, an einer Längsseite des Gehäuses (1), gegenüber den Aufnahmeelektroden (2, 3), angeordnete und sich über einen wesentlichen Teil der Länge des Gehäuses (1) erstreckende Grafikanzeigeeinheit (4) und ein Ein/Aus-Schalter (6) angeordnet und elektrisch mit der Diagnoseelektronik verbunden sind und die Diagnoseelektronik ferner eine Auswerteelektronik sowie eine Ansteuerelektronik für die Grafikanzeigeeinheit (4) umfaßt und wobei durch Aufsetzen des Gehäuses (1) auf einem Körperbereich die Aufnahmeelektroden (2, 3) an die Haut kontaktierend andrückbar sind und über die Aufnahmeelektroden die Herzfrequenz, ein Elektrokardiogramm (EKG), die QRS-Durchgänge eines EKG, ein Elektroenzephalogramm (EEG) od. dgl. aufnehmbar ist, **dadurch gekennzeichnet,** daß das Gehäuse (1) röhrenförmig, etwa füllhalterartig geformt ist und daß die eine Aufnahmeelektrode (3) als vom zugehörigen Ende des Gehäuses (1) ausgehender Halteclip ausgeführt ist.

2. Herzdiagnosegerät nach Anspruch 1, dadurch gekennzeichnet, daß das Gehäuse (1) eine Länge von 100 mm bis 160 mm, insbesondere von ca. 130 mm, und einen Durchmesser bzw. einen maximalen Durchmesser von 10 mm bis 40 mm, insbesondere von ca. 16 mm, aufweist.

3. Herzdiagnosegerät nach Anspruch 1, dadurch gekennzeichnet, daß die Auflösung der Graphikanzeigeeinheit (4) automatisch nach Maßgabe der maximalen Amplitude der Herzaktion einstellbar ist.

4. Herzdiagnosegerät nach Anspruch 3, dadurch gekennzeichnet, daß in der Graphikanzeigeeinheit (4) eine der gewählten bzw. gegebenen Auflösung entsprechende Referenzanzeige (9), insbesondere ein 1 mV/-Block, vorgesehen ist.

5. Herzdiagnosegerät nach Anspruch 1, wobei ein auf der Graphikanzeigeeinheit (4) angezeigtes EKG wahlweise festhaltbar ist (Einfrieren der Anzeige), dadurch gekennzeichnet, daß das Einfrieren der Anzeige automatisch erfolgt, und zwar bei Abnahme der Aufnahmeelektroden (2, 3) von der Haut eines Patienten nach einer durchgeführten Aufnahme.

6. Herzdiagnosegerät nach Anspruch 5, dadurch gekennzeichnet, daß das Wiederaufsetzen der Aufnahmeelektroden (2, 3) auf die Haut eine Löschung des festgehaltenen EKG und eine Fortführung der Aufnahme auslöst.

7. Herzdiagnosegerät nach Anspruch 5, dadurch gekennzeichnet, daß als Auslösekriterium der Bahnwiderstand zwischen den Aufnahmeelektroden (2, 3) dient, insbesondere bei einem Bahnwiderstand unter 3 kΩ eine Aufnahme des EKG und über 3 kΩ ein Festhalten des EKG erfolgt.

8. Herzdiagnosegerät nach Anspruch 6, dadurch gekennzeichnet, daß als Auslösekriterium der Bahnwiderstand zwischen den Aufnahmeelektroden (2, 3) dient, insbesondere bei einem Bahnwiderstand unter 3 kΩ eine Aufnahme des EKG und über 3 kΩ ein Festhalten des EKG erfolgt.

9. Herzdiagnosegerät nach Anspruch 1, dadurch gekennzeichnet, daß erforderliche Schalter (6, 8, 10, 17) als Schiebeschalter ausgeführt und in Längsrichtung des Gehäuses (1) angeordnet bzw. ausgerichtet sind.

10. Herzdiagnosegerät nach Anspruch 1, dadurch gekennzeichnet, daß an einer Stirnseite des Gehäuses (1) eine Diagnoseleuchte (7) angeordnet ist.

## Claims

1. A cardiac diagnosis device with an elongated case (1), diagnosis electronics disposed in the case (1) and two recording electrodes (2, 3) disposed on a longitudinal face of the case (1) near the front and rear end face, respectively, of the case (1) and electrically connected to the diagnosis electronics, wherein a power supply, a thin flat graphic display unit (4) disposed on a longitudinal face of the case (1) opposite the recording electrodes (2, 3) and extending over a significant part of the length of the case (1), and an on/off switch (6) are also disposed in the case (1) and electrically connected to the diagnosis electronics, and wherein the diagnosis electronics further comprise an electronic evaluation unit and an electronic controller for the graphic display unit (4), and wherein by placing the case (1) on a body region the recording electrodes (2, 3) can be pressed against the skin in contact therewith, and the cardiac frequency, an electrocardiogram (ECG), the QRS complexes of an ECG, an electroencephalogram (EEG) or the like can be recorded via the recording electrodes, characterised in that the case (1) is of tubular shape, somewhat like a fountain pen, and that one recording electrode (3) is constructed as a holder clip starting from the associated end of the case (1).

2. A cardiac diagnosis device according to claim 1, characterised in that the case (1) has a length of 100 mm to 160 mm, particularly of about 130 mm, and a diameter or a maximum diameter of 10 to 40 mm, particularly of about 16 mm.

3. A cardiac diagnosis device according to claim 1, characterised in that the resolution of the graphic display unit (4) can be adjusted automatically according to the maximum amplitude of the cardiac activity.

4. A cardiac diagnosis device according to claim 3, characterised in that a reference display (9) corresponding to the selected resolution, particularly a 1 mV block, is provided in the graphic display unit (4).

5. A cardiac diagnosis device according to claim 1, wherein an ECG displayed on the graphic display unit (4) can be optionally retained (freezing of the display), characterised in that freezing of the display is effected automatically when the recording electrodes (2, 3) are removed from the skin of a patient after making a recording.

6. A cardiac diagnosis device according to claim 5, characterised in that replacement of the recording electrodes (2, 3) on the skin triggers the deletion of the retained ECG and the continuation of the recording.

7. A cardiac diagnosis device according to claim 5, characterised in that the track resistance between the recording electrodes (2, 3) serves as the triggering criterion, and in particular that recording of the ECG occurs at a track resistance less than 3 kΩ and retention of the ECG occurs above 3 kΩ.

8. A cardiac diagnosis device according to claim 6, characterised in that the track resistance between the recording electrodes (2, 3) serves as the triggering criterion, and in particular that recording of the ECG occurs at a track resistance less than 3 kΩ and retention of the ECG occurs above 3 kΩ.

9. A cardiac diagnosis device according to claim 1, characterised in that the requisite switches (6, 8, 10, 17) are constructed as sliding switches and are disposed or aligned in the longitudinal direction of the case (1).

10. A cardiac diagnosis device according to claim 1, characterised in that a diagnosis lamp (7) is disposed on an end face of the case (1).

## Revendications

1. Appareil pour le diagnostic cardiaque, comprenant un logement (1) étiré en longueur, un dispositif électronique pour le diagnostic disposé dans le logement (1) et deux électrodes d'enregistrement (2, 3) disposées sur un côté longitudinal du logement (1) à proximité de la face frontale avant ou arrière du logement (1) et reliées électriquement au dispositif électronique de diagnostic, dans lequel, dans le logement (1), sont disposés, en outre, une alimentation en tension, une unité d'affichage graphique (4) plane, mince, disposée sur le côté longitudinal du logement (1) à l'opposé des électrodes d'enregistrement (2, 3) et s'étendant sur une partie importante de la longueur du logement (1), ainsi qu'un interrupteur (6) de mise en circuit/mise hors circuit, tout en étant reliés électriquement au dispositif électronique de diagnostic, ledit dispositif électronique de diagnostic englobant, en outre, un dispositif électronique d'évaluation, ainsi qu'un dispositif électronique de commande pour l'unité d'affichage graphique (4), et dans lequel, en appliquant le logement (1) sur un endroit du corps, les électrodes d'enregistrement (2, 3) peuvent être pressées pour venir se mettre en contact avec la peau; à l'intervention des électrodes d'enregistrement, on peut enregistrer le rythme cardiaque, un électrocardiogramme (ECG), les passages QRS d'un ECG, un électroencéphalogramme (EEG) ou analogues, caractérisé en ce que le logement (1) est façonné en forme tubulaire approximativement à la façon d'un porte-plume réservoir, et en ce que la première électrode d'enregistrement (3) est réalisée en forme de pince de fixation en partant de l'extrémité correspondante du logement (1).

2. Appareil pour le diagnostic cardiaque selon la revendication 1, caractérisé en ce que le logement (1) présente une longueur de 100 mm à 160 mm, en particulier d'environ 130 mm, et un diamètre ou un diamètre maximal de 10 mm à 40 mm, en particulier d'environ 16 mm.

3. Appareil pour le diagnostic cardiaque selon la revendication 1, caractérisé en ce qu'on peut régler automatiquement la résolution de l'unité d'affichage graphique (4) en fonction de l'amplitude maximale de l'activité cardiaque.

4. Appareil pour le diagnostic cardiaque selon la revendication 3, caractérisé en ce que, dans l'unité d'affichage graphique (4), on prévoit une indication de référence (9) correspondant à la résolution choisie ou fournie, en particulier un bloc de 1 mV.

5. Appareil pour le diagnostic cardiaque selon la revendication 1, dans lequel on peut fixer sélectivement un ECG affiché sur l'unité d'affichage graphique (4) (figeage de l'affichage), caractérisé en ce que le figeage de l'affichage a lieu de manière automatique, en fait, lorsqu'on retire de la peau d'un patient les électrodes d'enregistrement (2, 3) après avoir procédé à un enregistrement.

6. Appareil pour le diagnostic cardiaque selon la revendication 5, caractérisé en ce qu'une nouvelle application des électrodes d'enregistrement (2, 3) sur la peau provoque un effacement du ECG fixé, ainsi que la poursuite de l'enregistrement.

7. Appareil pour le diagnostic cardiaque selon la revendication 5, caractérisé en ce que, fait office de critère de résolution, la résistance en volume entre les deux électrodes d'enregistrement (2, 3); en particulier, avec une résistance en volume inférieure à 3 kΩ, un enregistrement du ECG se produit et au-delà de 3 kΩ, on obtient une fixation du ECG.

8. Appareil pour le diagnostic cardiaque selon la revendication 6, caractérisé en ce que, fait office de critère de résolution, la résistance en volume entre les deux électrodes d'enregistrement (2, 3); en particulier, avec une résistance en volume inférieure à 3 kΩ, un enregistrement du ECG se produit et au-delà de 3 kΩ, on obtient une fixation du ECG.

9. Appareil pour le diagnostic cardiaque selon la revendication 1, caractérisé en ce que les interrupteurs requis (6, 8, 10, 17) sont réalisés en forme d'interrupteur à coulisse et sont disposés ou alignés en direction longitudinale du logement (1).

10. Appareil pour le diagnostic cardiaque selon la revendication 1, caractérisé en ce que, sur une face frontale du logement (1), est disposée une lampe (7) pour le diagnostic.
